# EUROPEAN PATENT APPLICATION

(11) **EP 1 630 549 A1**
(43) Date of publication of application: **01.03.2006**
(21) Application number: 04447195.1
(22) Date of filing: 26.08.2004
(51) Int. Cl.: G01N 21/87, G01N 33/38

(54) **Method for gemstone tracing**

(71) Applicant: Jacobs, Ivo, 2610 Wilrijk (BE)
(72) Inventor: Jacobs, Ivo, 2610 Wilrijk (BE)
(74) Representative: Brants, Johan P.E.

(57) **Abstract**

The present invention relates to a method for tamperproof relating a gemstone originating from a rough stone to said rough stone, comprising the steps of (i) assigning to said rough stone a parental Idₚ; (ii) cleaving or sawing a table facet to said rough stone; (iii) assigning a first descendant ID_{d} to said rough stone provided with a table facet; (iv) processing said rough stone provided with a table facet into a gemstone; (v) assigning a further descendant ID_{d} to said gemstone; and (vi) tamperproof relating each descendant ID_{d} mutually and to the parental IDₚ.

The present invention further relates to a method for tamperproof relating at least two gemstones originating from one rough stone to said rough stone, comprising the steps of (i) assigning to said rough stone a parental IDₚ; (ii) splitting, e.g. by cleaving or by sawing, said rough stone in at least two parts for further processing into at least two gemstones, thereby forming a table facet to each of the at least two parts; (iii) assigning a first descendant ID_{d} to each of said at least two parts; (iv) processing said at least two parts into at least two gemstones; (v) assigning a further descendant ID_{d} to each of said at least two gemstones; and (vi) tamperproof relating each descendant ID_{d} mutually and to the parental IDₚ.

## Description

### Field of the Invention

The present invention relates to a tamperproof or fraud proof tracing system for diamonds and other gemstones, and in particular to a tracing system with a record of identity of origin from the original parent rough stone to one or more descendant cut or finished stone(s) originating from the same parental rough stone.

### Background

Since ages, the purchase of gemstones and, in particular, diamonds typically stands for the ultimate symbol of mutual love and commitment. The diamond which originated hundreds of millions of years ago, deep under the earth's crust is characterized by nature by its specific form. As such the octahedron represents about 30% of all natural minded diamond which is most often split in two or more parts.

Presenting two or more stones that are cut from the same rough stone provides the end purchaser with higher emotional value, e.g. such as engagement and wedding rings; and manufacturers are provided with a higher value of the rough gemstone material. This principal for enhanced marketing methods is disclosed in e.g. CA 2 343 448.

The value of such marketing strategy, however, resides in an incontestable furnished proof of origin of the finished stones.

WO 02/10091 discloses a method for marking and identifying gemstones, mined materials or objects, precious metals, or other similar valuable materials by encasing selected gemstones within removable casing. Identifiers may be included within the casing or actually branded directly onto the mined objects. The problem solved in WO 02/10091 is the provision of a method to secure a gemstone and document information such as its genealogy, origin, and chain of custody, quality attributes, ownership information, and other types of information, which does not degrade the gemstone, by accepted standards. Hereto, minded gemstones are encased in a removable polymeric substance, wherein the encasement can be further fingerprinted. Before or after the encasement is removed, an identifier branded onto the gemstone or onto the encasement may be read by a reader or scanner. Finished or cut gemstones may be similarly encased.

A disadvantage of the method according to WO 02/10091 is that the identification/tracing code applied to a mined "rough" or finished gemstone is not inseparable linked to the stone. Therefore, this identification/tracing code is lost from the (finished) stone once it becomes separated or removed from its encasing. Also in the case of branding an identifier directly on the minded stone, this identifier will be lost from the stone once it will be further processed to a finished stone.

Also CA 2 325 130 presents a method for tracing rough diamonds from the mine site to the finished product and the end-purchaser for the purpose of providing a "green diamond" with an effective audit trail. In CA 2 325 130, a tracing serial number or marking is laser engraved in the rough diamond crystals and this record is then associated with e.g. a Gemprint™ certificate number of the eventual finished cut stone for the identification of the finished cut stone as "stone with provenance".

The prior art methods thus provide tracing systems for gemstones and diamonds employing externally introduced identifiers to the rough stones or to the finished stones. Such externally introduced identifiers are of no value in the proof of origin of the stone and are prone to fraudulent practices, as they are easily removable from the stone onto which the identifier was placed. The coding is not related to the intrinsic characteristics of the stone.

The present invention aims at providing a method for the tamperproof identification/tracing of at least one cut and/or finished gemstone or diamond descending from one rough stone wherein the identifier is intrinsically linked to the stone(s) and traceable, not only throughout the procedure of processing steps from rough to finished stones, but also traceable throughout the stone's lifetime - the coding is an intrinsic characteristic of the stone itself. Accordingly, not only the economic value of the rough and finished stone(s) but also the emotional value to the end-purchaser of the finished stone(s) will be increased.

### Summary of the Invention

It is an object of the present invention to provide a fast and reliable tracing system for at least one cut or finished gemstone to its original rough stone. The present methods provide a tamperproof identification/tracing of at least one gem from a rough wherein the at least one first descendant stone(s) as directly derived from splitting or cleaving the rough are given an identity making them traceable already from the earliest stage in processing to finished gem(s).

In particular, present invention provides a method for tamperproof relating a gemstone originating from a rough stone to said rough stone, comprising the steps of (i) assigning to said rough stone a parental Idₚ; (ii) cleaving or sawing a table facet to said rough stone; (iii) assigning a first descendant ID_{d} to said rough stone provided with a table facet; (iv) processing said rough stone provided with a table facet into a gemstone; (v) assigning a further descendant ID_{d} to said gemstone; and (vi) tamperproof relating each descendant ID_{d} mutually and to the parental IDₚ.

Present invention further provides a method for tamperproof relating at least two gemstones originating from one rough stone to said rough stone, comprising the steps of (i) assigning to said rough stone a parental IDₚ; (ii) splitting, e.g. by cleaving or by sawing, said rough stone in at least two parts for further processing into at least two gemstones, thereby forming a table facet to each of the at least two parts; (iii) assigning a first descendant ID_{d} to each of said at least two parts; (iv) processing said at least two parts into at least two gemstones; (v) assigning a further descendant ID_{d} to each of said at least two gemstones; and (vi) tamperproof relating each descendant ID_{d} mutually and to the parental IDₚ.

Present invention has the advantage to known methods in the art that the identifiers or IDs are intrinsically linked to the stones including the parental rough stone and the cleaved, bruted, blocked, further, facetted (four-facet-stage; eight-facet-stage, etc.), polished and finished descendants. In the methods according to the present invention, further descendant ID's may be assigned, to the parts which are derived from a rough and processed to a finished gem, after each processing step or, alternatively, after several processing steps whereby some processing steps are not accompanied with an identification of the further processed gem. In the methods according to the present invention the ID's (IDₚ and first and further ID_{d}) are fast and easily related to ID's assigned in former or later processing steps so as to assure their origin throughout the procedure of making at least one finished stone or gemstone from a single rough stone without the need of time-consuming handling steps.

### Detailed Description of the Invention

The practice of the present invention will employ, unless otherwise indicated, conventional techniques which are within the skill of the art.

In this specification and the appended claims, the singular forms "a", "an", and "the" include plural references unless the context clearly dictates otherwise.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred devices, methods and materials are now described.

The present invention is directed to methods for uniquely relating, in a non-invasive manner, to the original parental rough stone, at least one gemstone, or at least one part derived from said rough stone to be processed into at least one gemstone, wherein the conduct of proof of the tamperproof relation is based on the intrinsic external and internal characteristics of the concerning stones including rough stone, finished gemstone(s) and any intermediary stone(s).

As used within the present specification, the terms "rough stone", "rough gem", "rough material", "parental stone" or "parental rough stone" are used interchangeably and refer to a precious mined crystalline rock or stone which has not yet undergone any further processing. A rough stone therefore is not split, cleaved, sawn, laser-treated, cut, or processed in any other way. Such rough stone may also be referred to as a "makeable".

As used within the present specification, the terms "finished stone", "finished gemstone", and "finished gem" are used interchangeably and relate to a stone which is fully processed and has undergone all processing steps for it to be ready for the jeweller to use.

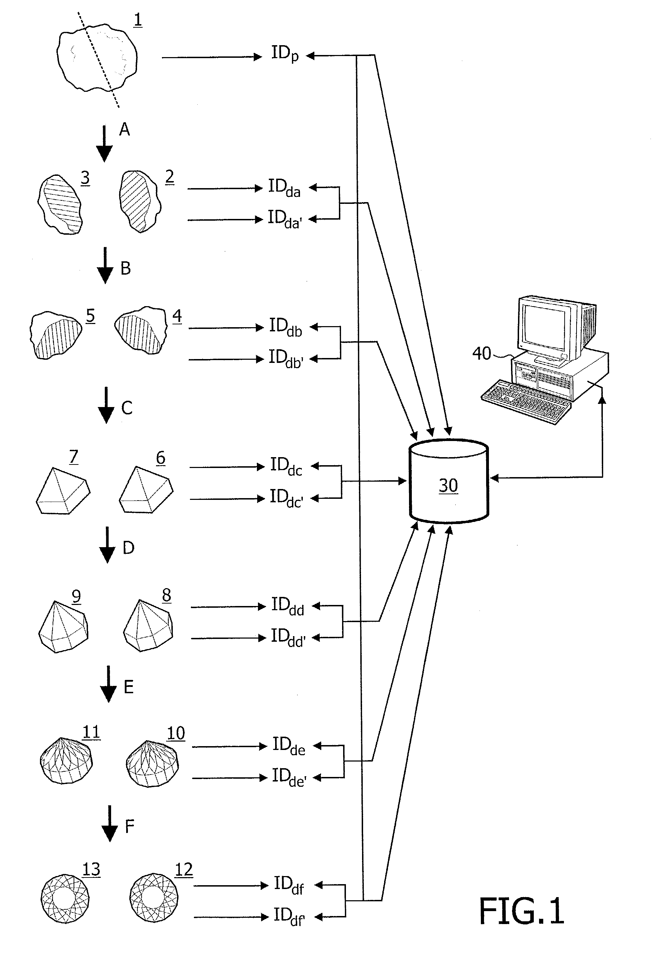

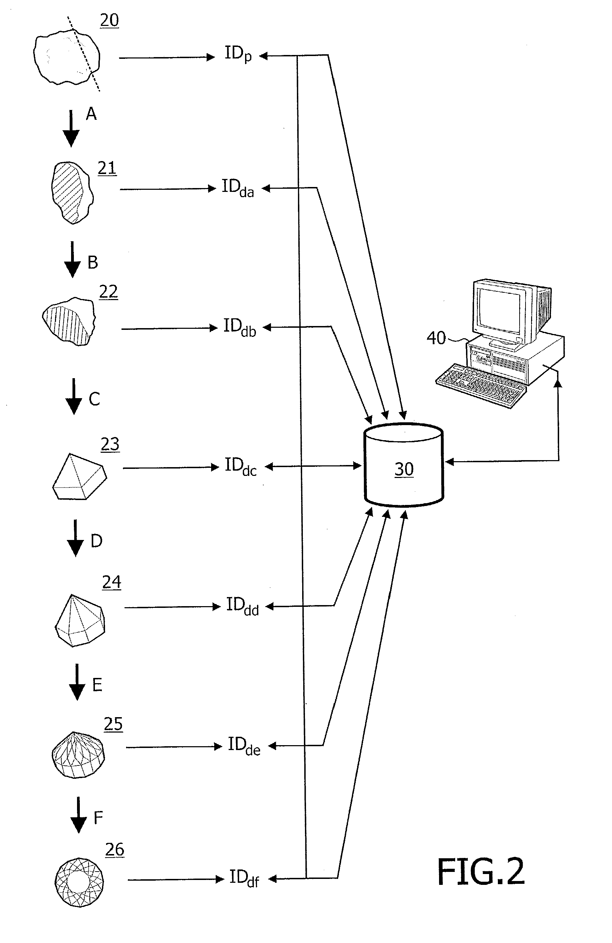

As used within the present specification, the terms "part derived from a rough stone", "split part", "rough stone provided with table facet", "intermediary gem", "gem" or "gemstone" are used interchangeably and refer to a precious crystalline rock or stone of any kind which is split or cleaved from a rough stone or which is derived subsequent to table formation of the rough. A "part derived from a rough stone" which includes also the part derived from the rough stone immediately after table placement, or "rough stone provided with table facet", may have undergone one or more further processing steps or not. A gemstone as used within the present specification refers to any intermediary stone in the processing of a rough stone to one or more finished gemstones. Typically, gems which are derived immediately after splitting or cleaving the rough stone or after table formation (step A in Figures 1 and 2) and which have not yet undergone any further processing are referred to as "first descendant gems".

As used within the present specification, the term "parental IDₚ" or "IDₚ" refers to the identification which is assigned to the rough stone.

As used within the present specification, the term "first descendant ID_{d}" or "first ID_{d}" refers to the identification assigned to the first descendant gems or the parts derived immediately after the first processing step (step A in Figures 1 and 2), i.e. placing the table facet to the rough. Where two or more finished gems are to be made from a rough, the rough will be split, by cleaving, sawing or laser treatment, whereby each first descendant part, as a result from the splitting of the rough, will be provided with a table facet.

As used within the present specification, the term "further descendant ID_{d}" or "further ID_{d}" refers to the identification assigned to the descendant gems which are obtained as from the completion of the second processing step (step B in Figures 1 and 2) including the finished gems.

The conduct of proof according to the present invention is based on the intrinsic external and internal characteristics of the concerning stones and as such reaches trough the entire processing from rough stone to finished gemstone.

Non-limiting examples of stones suitable in the methods according to the present invention are amethyst, aquamarine, beryl, diamond, emerald, garnet, kunzite, opal, orothoclase, peridot, ruby, sapphire, spinel, sunstone, tanzanite, topaz, tourmaline, quartz, and zircon.

### Splitting of rough stones into at least one part and further processing into one or more finished gemstone(s)

### 1. Splitting, cleaving, sawing or laser-treatment

If the rough material is of poor shape, or if it has conspicuous defects in it which prevent its being made into a single stone, it is split, e.g. by cleavage, sawing or by laser treatment, along its grain. In the case of diamond, hard as it is, it splits readily in certain definite directions (parallel to any of the triangular faces of the octahedral crystal). The cleaver has to know the grain of rough gems from the external appearance, even when the crystals, as found, are complicated modifications of the simple crystal form. He can thus take advantage of the cleavage to speedily reduce the rough material in size and shape to suit the necessity of the case. The rough material can be cleaved along its grain in two parts, each part thereby having one table facet, or can be used as basis for a single gemstone. In the latter case, the rough is not split and a table facet is placed onto the rough stone by cleaving along its grain.

### 2. Bruting

The next step is to give the rough material a shape closely similar to that of e.g. a finished brilliant but rough and without facets. After the receipt of cleaved, sawn or laser-treated stones which are provided with a table facet, they are individually planned so as to ensure the maximum yield, and better quality of the finished product. After the rough stone has been planned, it is then shaped or cut by placing the rough stone in the end of a holder by means of a tough cement and then holder and stone are rotated at high speeds in a lathe-like machine. The sharp edges are rounded off according to the plan of the stone; the stone has a top and a bottom. In certain cases, this bruting process wherein the shape and position of the facets are roughly marked out is done by double spindle instead of lathe for better results. In the case of diamonds, another rough diamond may be cemented into another holder and held against the surface of the rotating diamond. The holder is steadied against a firm support. It now becomes a case of "diamond cut diamond," each stone wearing away the other and being worn away itself. The stones which do not have the required depth are rebruted.

### 3. Blocking - four-facet-stage

The bruting process is fairly rapid and it leaves the stone (which is reversed to make the opposite side) round in form and with a rounding top and cone shaped back. The facets must now be polished onto the stone.

The largest facet of the gem, called the "table", is the most important since it is through this facet that the entire brilliance of the finished gem is displayed. The polishing of this crucial facet, called table polishing is done according to the plan of the gem. After the girdle has been given a circular shape, the stone is fixed in collect type pots and wass-covers according to the stone's diameter and depth, for bottom polishing. The grains of the natural surface are identified and set in a predetermined direction before polishing the lower half of the stone. In the blocking process, the lower half of the stone is given four facets. Polishing of four main facets is done on the bottom, facilitating the further processing of the gem. This helps in improvement of productivity.

### 4. Eight-facet-stage

Subsequent to the blocking, a diamond destined to become a brilliant is given eight facets to its lower half.
In the classic "brilliant" design, each stone is finally given 57 facets, 32 facets on the top of each stone, 24 facets on the bottom, and 1 table facet.

### 5. Finished gem

After the facets are cut, the stone is thoroughly checked to detect any damage or mistake and is finally boiled in various types of acids in a conditioned environment to clean it. Typically polished gems of 30pts and less are re-assorted in marketable lots keeping in view the market demand, realizable price and customer preferences. Larger stones (>30pts) are offered mostly with a separate certificate.

Accordingly, processing a rough stone to one or more finished gemstones relates to a step-wise process comprising several well-defined stages such as planning, cutting, bruting, faceting and polishing.

Accordingly, in one embodiment of the present invention, methods for uniquely relating one or more gemstones originating from one rough stone are provided, wherein said processing comprises the steps of, bruting, blocking, further faceting and polishing.

Present invention provides incontestable conduct of proof characterized in that completion of each stage or step in the entire processing of rough stone to finished gemstone may be accompanied by assigning a descendant ID_{d} to the part(s) derived from same rough stone and relating said ID_{d} with any ID_{d} assigned in any former or later stage or processing step and ultimately also to IDₚ in the same process. Alternatively, assigning further descendant ID_{d}'s may also be completed after completion of several processing steps.

Accordingly, in a further embodiment, a method according to the present invention is provided, wherein the assigning of a further descendant ID_{d} is subsequent to several processing steps.

Accordingly, in a further embodiment, a method according to the present invention is provided, wherein the assigning of a further descendant ID_{d} is subsequent to each processing step.

### Identification of rough stone - parental IDₚ

As described above, after a rough diamond or other rough gemstone has been found it goes through many processing stages before it becomes the finished product we are familiar with. The first step in manufacturing a diamond or other gemstone is to place the table facet or, alternatively, split it by cleaving, sawing, or laser treatment - that is to cut or cleave the rough stone in at least two pieces in order to bring out the best profile that will determine the end product of the stone; i.e. will it be pear shape or a marquise or a round, etc. In the latter case, each of the parts derived from the split rough is thereby also provided with a table facet.

The decision to split or not, and how to split the rough stone while achieving the maximum yield possible is based on the stone's crystal form or crystal lattice which is the so called intrinsic external characteristic of the stone. The most common shape of for example rough diamonds is the octahedron which is well-suited for splitting into two descendant parts for further processing. This crystal form with eight faces resembles two pyramids joined at their base. Diamonds are most frequently cut in the brilliant form, which form is comparable to such octahedron of which the two opposite corners have been truncated. A truncated octahedron, therefore, can be transformed into a brilliant by simply adding the necessary facets; hence the form of crystal, which can be most conveniently cut as a brilliant, is the octahedron. The rhombic dodecahedron and the hexakis-octahedron are also suitable; but stones, whose form differs widely from that of the regular octahedron, cannot be so easily transformed into brilliants. Before such a stone is faceted it is reduced by cleavage to the octahedral form, in order to avoid the tedious process of grinding away portions, which need to be removed. The property of cleavage then is very useful to the diamond-cutter, for not only is he spared much labour in grinding, but the fragments removed by cleavage can be utilized in the fashioning of smaller gems; moreover, the property can be made use of for the purpose of removing the faulty portions of a stone or of dividing a large stone of unsuitable form into several smaller ones.

The direction of cleavage, or grain, and the different shape possibilities are thus determined by the outward form of the rough stone or crystalline structure including location of imperfections and internal flaws (structural defects), further herein referred to as the "crystal map" which is unique to each rough stone. After placing the table facet to a rough stone, each first descendant stone will therefore have the crystal map corresponding to a certain fraction of the parental or rough crystal map. In the case of splitting the rough stone in at least two parts, only the crystal maps of those first descendant parts from same rough stone will fit together to obtain again the original parental crystal map.

Therefore, the rough stone crystal map provides a unique signature which allows split and non-split first and further parts to be traced back to the parental rough stone. In the case of splitting the rough stone in at least two parts, this is by amalgamating the descendant crystal maps.

Stone crystal maps may be detected and identified by spectroscopy or incident radiation systems. Various incident radiation measurements may provide data which may become visualized through one of the numerous possible output signals such as 3D structure graphics. In this respect, DiaExpert™ is mentioned which refers to a laser scanning technology allowing mapping of grooves, holes and concave areas on the rough stone represented by a graphical printout of the crystal map.

As parts derived from rough advance through the different stages of processing, individual crystal plans will each loose to a certain degree their integrity as each processing step comes along with loss of material and hence a partial destruction of the original crystal structure. As a result, comparison or amalgamation of the crystal plan(s) of finished gem(s) will usually no longer come up to the exact original parental crystal plan. The closer the original crystal plan is to the sum of the individual descendant crystal plans, the less difficult is a tracing based on crystal structures.

In contrast to the loss of external characteristics during processing, partial loss of internal characteristics at the gem's surface does not count against identity relation based on the most inner characteristics of stones in a tracing procedure and reliability is incontestable. The term "inner characteristic" or "internal characteristic" relates to inclusions and birthmarks of a gem (rough, intermediary or finished) including cleavage cracks and feathery marks which often mar the transparency of the gem, and these may appear as black spots when seen at an angle such that light is totally reflected at their surfaces. Inclusions are common in diamonds and occur as small crystals enclosed by the diamond lattice. The most common inclusions are magnesian olivine, haematite, magnetite, pyrope garnet, enstatite, zircon, diopside, rutile, pyrrhotite, and liquid carbon dioxide. The pyrope garnet inclusions are unusually rich in chromium. Diamond itself is occasionally found as an inclusion in diamonds, but graphite is rarely found. Quartz has been found included only in Brazilian diamonds.

If the internal characteristics of a gem would be compared to a starry sky, then, as a part derived from a rough is processed through a series of processing steps, the centre of the starry sky will always remain, despite the loss of some characteristics which were placed at the edges. Therefore partial loss of internal characteristics at the gem's surface (edge) does not count against identity relation based on the most inner characteristics of stones in a tracing procedure according to the present invention.

Intrinsic internal characteristics may be determined by various techniques, in particular by incident radiation systems.

Accordingly, in one embodiment of the present invention, methods for uniquely relating one or more gemstones originating from one rough stone are provided, wherein parental IDₚ and first and further descendant ID_{d}'s are assigned by an incident radiation system.

### Identification of gemstones and finished gems - descendant ID

Identification systems of gemstones are ample and may for example relate to incident radiation systems.

Spectroscopy in general allows measurements of various properties (absorption, transmittance, and luminescence) of a material, usually depending on wavelengths/energy. The sampling of measurements is then visualised through various output signals such as e.g. plotted in a graph. Ultra violet, visible, near infra red, infra red, Raman, photoluminescence and cathodoluminescence are the most important spectroscopic methods in advanced gemstone testing/identification.

With UV/VIS/NIR spectroscopy, complete spectra can be obtained in the range of 190-1000nm. It is basically measured how much light is absorbed or transmitted at what wavelength by a substance (here a gemstone) and this is then plotted in a graph. The spectral curves obtained give a lot of information about the colour of diamonds. Most colour centres (defects in the crystal lattice which cause colour) are detected by this technique.

With liquid nitrogen immersion, a gem is held in a special cell immersed in liquid nitrogen, which has a temperature of -196°C. The extreme cooling is necessary to ensure the highest possible resolution of all absorption-features in UV/VIS/NIR- and Raman/photoluminescence-spectroscopy. Rarely, liquid helium (-269°C) instead of nitrogen, is employed.

With FTIR or IR spectroscopy, absorption is measured from 800-25.000 nm. This is especially useful to determine gem/diamond types and/or impurity concentrations.

With Raman-photoluminescence, a low-power laser of various wavelength (e.g. 405, 514, 532 nm) is focused on the gemstone. Optical centres are excited by the laser and emit a measurable amount of luminescence. These luminescence phenomena appear as peaks in the spectrum. Photoluminescence detects certain colour centres with much more sensitivity than other spectroscopic methods.

With Raman-(micro) spectrometry, a back-scattering technique where a low-power laser of various wavelength (e.g. 405, 514, 532 nm) is focused on a gem and some of this laser-light is shifted to a longer wavelength and re-emitted by the stone. This so called "Stokes radiation" appears as peaks in the spectrum. These peaks are characteristic for the gem. The microspectrometry technique is useful to identify most inclusions in gemstones, which do not necessarily have to reach the surface. Here, the laser is focused through a microscope and identifies small inclusions as small as one micrometer.

Cathodoluminescence is somewhat similar to photoluminescence, but instead of a laser beam, an electron beam is being used to excite the optical centres. Cathodoluminescence can be either used spectroscopically or as an observative technique where the colour and the pattern of the luminescence can be observed.

With scanning electron microscopy (SEM), electrons hit the surface of the stone by which they are back scattered and then magnified and focused by various lenses. This allows magnifications up to 2.000.000x, but only of surfaces; images of internal characteristics are not possible. Most SEM instruments also have Electron Microprobe Analysis (EMPA) available, with which chemical analysis of substances is possible and which can detect certain impurities in diamonds

### Tamperproof relating one or more gemstones originating from one rough stone to said rough stone

Identification systems of rough stones and (finished) gemstones are ample and may relate to incident radiation systems comprising optical, electromagnetic and also acoustic radiation. Incident radiation systems allow identification of gems according to their intrinsic outer and/or inner characteristics. Typically, the intrinsic outer or external characteristic of a gem is represented by the crystal form or structure (lattice) or the crystal plan if location of imperfections and internal flaws (structural defects) are taken into account. Typically, the intrinsic inner or internal characteristic of a gem is represented by all imperfections in the crystal structure of the gem as well as to imperfections of the polished surfaces of the same.

Accordingly, in one embodiment of the present invention, methods for uniquely relating one or more gemstones originating from one rough stone are provided, wherein a radiation is chosen from the group comprising optical, electromagnetic and acoustic radiation.

Methods according to the present invention preferentially employ light especially instead of other forms of energy.

Accordingly, in a further embodiment of the present invention, methods for uniquely relating one or more gemstones originating from one rough stone are provided, wherein radiation is optical.

The methods according to the present invention allow deducing a tamperproof relationship between a rough stone and its descendants. Assigning IDs may involve employment of one or more optical scanning systems. The choice of identification system may depend on the stone type, i.e. rough, intermediary, or finished. For example, identification of a rough gem according to its crystal structure or crystal plan provides an excellent and unique IDₚ to the stone. However, as a rough gem undergoes subsequent processing steps, its outer structure or the outer structure of its descendants will be, at least partially, lost, making it more difficult to trace the rough based on crystal structure or lattice alone.

Optical scanning systems which allow the identification of gems based on detection of internal optical centres in the stone provide for measurements that are not influenced by any loss of external characteristics such as partial destruction of crystal lattice. As such, an optical scanning system which provides data on a gem's internal characteristics therefore allows relating, incontestably, a finished gem to each of its former intermediary forms including or not the rough stone.

In the methods according to the present invention, reliability of the tracing method is optimal guaranteed through an IDₚ assignment to the rough stone which differs from further ID_{d} assignment to the further descendant stones. The present methods may thus employ at least two identification systems based on optical scanning to the detection of gemstone characteristics being either focussed on the stone's intrinsic external or internal characteristics.

Accordingly, in a further embodiment of the present invention, methods for uniquely relating one or more gemstones originating from one rough stone are provided, wherein the parental IDₚ is assigned by an optical scanning system which is different from the optical scanning system in the assignment of the first and further descendant ID_{d}'s.

In yet a further embodiment of the present invention, methods for uniquely relating one or more gemstones originating from one rough stone are provided, wherein the first descendant ID_{d}'s are assigned by a first optical scanning system which is equal to the optical scanning system in the assignment of the parental IDₚ and by a second optical scanning system which is equal to the optical scanning system in the assignment of the further descendant ID_{d}'s.

Thus, although conduct of proof in the unique and tamperproof relation between at least one descendant gemstone and the original rough stone may involve just one identification system throughout the processing, it is preferred that more than one identification system is employed covering the identification of external characteristics as useful in the identification of rough and first descendant stones as well as the identification of internal characteristics as most suitable in the identification of gems in the final and former intermediary stages of processing. Usually, in the methods according to the present invention, the first descendant gems (e.g. intermediary gems indicated by "2" and "3" in Figure 1, and intermediary gem indicated by "21" in Figure 2) undergo identification by two identification systems which work complementary, covering the intrinsic external as well as internal characteristics of this particular intermediary gem.

The difference in identification systems when opting for employment of more than one in identification system in a method according to the present invention usually resides in the difference of output signal. That is, measured data obtained by an optical scanning system, or other system, may be visualized as 3D-graphic for example in crystal structure/lattice reproduction, or a 2D plot for example in refraction/reflection data, or other.

Adequate optical scanning systems for identification of rough, intermediary as well as finished gems, regardless of the type of output signal, may employ light amplification by stimulated emission of radiation and may utilize the natural oscillations of atoms or molecules between energy levels for generating coherent electromagnetic radiation usually in the ultraviolet, visible, or infrared regions of the spectrum.

Accordingly, in one embodiment of the present invention, methods for uniquely relating one or more gemstones originating from one rough stone are provided, wherein the optical scanning system is laser scanning.

Identification of rough stones having unique crystal structures or crystal maps therefore preferentially are determined by an optical scanning system providing output signals as 3D structure graphics.

Accordingly, in a further embodiment of the present invention, methods for tamperproof relating one or more gemstones originating from one rough stone are provided, wherein first descendant ID_{d}'s are assigned by a first optical scanning system which is equal to the optical scanning system in the assignment of the parental IDₚ, and wherein said parental IDₚ is assigned by means of a laser scanning system producing a visual 3D output signal.

In this respect, DiaExpert™ is mentioned which refers to a laser scanning technology allowing mapping of grooves, holes and concave areas on the rough stone represented by a graphical printout of the crystal map.

Accordingly, in a further embodiment of the present invention, methods for uniquely relating one or more gemstones originating from one rough stone are provided, wherein DiaExpert™ is employed as a laser scanning system producing a visual 3D output signal.

With respect to intermediary and finished gems, most adequate identification systems are based on recording a plurality of spots resulting from the internal refraction and reflection of light arrays due to presence of so called optical centres such as for example imperfections in crystal structure and impurities.

Accordingly, in a further embodiment, methods according to the present invention are provided, wherein first descendant ID_{d}'s are assigned by a second optical scanning system which is equal to the optical scanning system in the assignment of the further descendant ID_{d}'s and wherein said further descendant ID_{d}'s are assigned by means of a laser scanning system producing an optical output of the internal refraction and reflection characteristics of the gemstones.

Reflection and refraction characteristics of a gemstone or finished gemstone, in particular a diamond, define a unique "sparkle" pattern for that particular gemstone, thus identifying the gemstone. Reflection and refraction characteristics of a gemstone or finished gemstone may be installed upon radiation of the gem with a low-powered laser. For example, Gemprint™ employs a low powered laser for diamond identification and identification of other gems of a high degree of brilliance.

Accordingly, in a further embodiment, methods according to the present invention are provided, wherein said means of refraction and reflection of light arrays is Gemprint™.

Gemprint Corporation markets a system and manages a database where the optical responses of gemstones are determined and the optical responses are recorded on a recording medium, preferably a photographic medium and stored in a database for future reference.

In conjunction with Gemprint Corporation's database, step-by-step processing data as obtained in the methods according to the present invention may be stored in an international genuine-gem-database to be accessed by retailers, jewellery shops, via e-commerce and also by wholesalers for e.g. manufacturers. End purchasers may be given an entrance code to access the unique ID number(s) of the purchased gems.

The increased economic as well as emotional value attached to gemstones emerging from a method according to the present invention may be laid down in a certified statement which may be offered in combination with the concerning gems. Such certificate may also include the above-mentioned entrance code.

It is therefore another object of the present invention to provide a certificate of authenticity attesting the tamperproof relationship of a gemstone to its original rough stone comprising proof by the method according to the present invention characterized in:
i. parental IDₚ of the rough stone;
ii. first descendant ID_{d} for the rough stone provided with table facet;
iii. further descendant ID_{d} for further processed rough stone provided with table facet and;
iv. attest of tamperproof relationship between each descendant ID_{d} mutually and the parental IDₚ.

It is to be understood by part (iii) of the above-mentioned certificate that the certificate is provided with one or more ID_{d} corresponding to the ID_{d}'s of each intermediary gem (or further processed gem) in the process from rough to finished gem or corresponding to the ID_{d}'s of some intermediary gems only. For example, in the case where an ID_{d} is assigned after completion of several processing steps, it is likely that the certificate will mention only the ID_{d}'s to some intermediary gems instead of the ID_{d}'s to all intermediary gems.

It is another object of the present invention to provide a certificate of authenticity attesting the tamperproof relationship of at least two parts from the same rough stone comprising proof by the method according to the present invention characterized in:
i. parental IDₚ of the rough stone;
ii. descendant ID_{d} for each of the at least two parts derived from splitting the rough stone;
iii. further descendant ID_{d} for each of the at least two parts derived from splitting the rough stone at some or each of the processing steps of the method according to the present invention; and
iv. attest of tamperproof relationship between the descendant ID_{d} of each of the at least two parts derived from the parental rough stone mutually and the parental IDₚ.

It is to be understood by the term "mutually" that each descendent ID assigned in the methods of the present invention can be compared to any descendent ID assigned in former or later steps in the same process and can be compared, naturally, also to the parental ID.

The present invention also provide a kit for presenting one or more gems originating from one rough stone.
Accordingly, it is a further object of the present invention to provide a kit comprising:
i. at least one gemstone derived from a rough stone, said at least one gemstone identified through a method according to the present invention; and
ii. a certificate according to the present invention.

An advantage of the methods according to the present invention is its value in discriminating so-called synthetics from genuine gems. The term "synthetic" as used herein is defined as having the composition of a known mineral, but does not necessarily match the composition of the gemstone it is to mimic. Synthetics may be created by mimicking the natural process, by recrystallizing natural stones, or through an entirely new man-made technology. Synthetics share the same chemical structure, and general physical properties with their natural counter parts but they are created in a laboratory.

For example, Apollo Diamond Inc. in Massachusetts and Florida-based Gemesis Corp. each have produced a new type of lab-made diamond that is difficult, though not impossible, to distinguish from natural diamonds.

Natural diamonds are mined from deep in the earth. They are formed of carbon that has been exposed to immense heat and pressure over billions of years. As a consequence, natural diamond shows a typical trigone structure. In contrast, the new manmade diamonds may be created in a few days by a machine through e.g. a process called chemical vapour deposition (Apollo Diamond Inc.). Diamond crystal is formed when a plasma cloud of carbon is deposited onto diamond wafers. The wafer seeds grow into diamond mini-bricks or rough diamonds that later on may sliced into wafers, and cut and polished into diamonds. Alternatively, synthetic diamonds may be created through a highpressure, high-temperature technique that mimics the geologic conditions under which natural diamonds are formed (Gemesis Corp.). In a capsule placed under high temperature and pressure, graphite ― a form of carbon ― breaks down into atoms and travels through a metal solvent to bond to a tiny diamond seed, crystallizing layer by layer.

Three or four days later, the stone that is formed is then removed from the chamber and cut and polished into a synthetic diamond.

The companies say that their manmade gems - to be sold at lower prices (up to 30%) than natural diamonds - will be labelled so that they will not be confused with natural diamonds.

Obviously, these manmade gems are not fraud-proof as the label may be easily removed and this is of highly concern in diamond trading and sales. However, the typical trigone structure present on a natural rough diamond will remain absent from the lab-made rough diamonds. Therefore, in a method according to the present invention, rough lab-made gems (diamonds), offered on the market in bad faith as genuine roughs will be readily identified as synthetic in the first identification, thereby excluding the synthetic from being further processed to finished gemstone and hence excluding such synthetic from being included in a genuine-gem-database.

A finished lab-made gemstone, therefore will never have provided with it a traceable proof of origin according to the present methods and will therefore be marked as non-genuine or synthetic.

Present invention therefore offers a highly valuable tool in excluding from the pool of natural diamonds any unnatural, synthetic or lab-made gem, rough or finished.

Accordingly, it is another object of the invention to provide for the use of a method according to the present invention and as described herein for proving genuineness of a gemstone.

### Short Description of the Figures

It is noted that the figures are a mere illustration of a method according to the present invention and not in any way limiting.

**Figure 1** illustrates a method according to the present invention wherein steps (A) to (F) present a number of processing steps from one rough (1) to at least two finished stones (10 to 13). One rough stone is given a parental IDₚ after which it is split in step (A) along its grain (dotted line) in at least two parts (2) and (3) for processing to at least two finished gems (10) to (13). Said at least two parts provided with a table facet after step (A) are given first descendant IDs (ID_{da} and ID_{da'}) which is then tamperproof related to the original parental IDₚ of the rough stone (1). In the present figure, steps (B) to (F) relate to the respective bruting, blocking (four-facet-stage), eight-facet-stage, and final polishing steps. After each or several processing steps a further ID_{d} and ID_{d'} is assigned to the at least two intermediary or finished gemstones, allowing each or several processing steps to be concluded with tamperproof relating descendant gems from same parental rough to intermediary and/or parental stones in the same process. Each ID assignment is recorded in a genuine-gem-database system (30) which is accessible by any party from a personal computer (40) for fast tracing of the tamperproof relationship between rough (1) and descendants (2) and (3), (4) and (5), (6) and (7), (8) and (9), (10) and (11); and/or (12) and (13) throughout the processing steps (A) to (F).

**Figure 2** illustrates a method according to the present invention wherein steps (A) to (F) present a number of processing steps from one rough (20) to one finished gem (26). One rough stone (20) is given a parental IDₚ after which it is cleaved, sawn or laser-treated in step (A) along its grain (dotted line) to obtain intermediary gem (21) provided with a table facet for further processing in one finished gem (26). Said intermediary gem (21) provided with a table facet after step (A) is given first descendant ID_{da} which is then tamperproof related to the original parental IDₚ of the rough stone (20). In the present figure, steps (B) to (F) relate to the respective bruting, blocking (four-facet-stage), eight-facet-stage, and final polishing steps. After each or several processing steps a further ID_{d} is assigned to the intermediary gem (22), (23), (24), and/or (25) or finished gem (26), allowing each or several processing steps to be concluded with tamperproof relating the descendant gem from a parental rough to intermediary and/or parental stones in the same process. Each ID assignment is recorded in a genuine-gem-database system (30) which is accessible by any party from a personal computer (40) for fast tracing of the tamperproof relationship between rough (20) and descendants (21), (22), (23), (24), (25); and/or (26) throughout the processing steps (A) to (F).

## Claims

1. Method for tamperproof relating a gemstone originating from a rough stone to said rough stone, comprising the steps of
i. assigning to said rough stone a parental IDₚ;
ii. cleaving or sawing a table facet to said rough stone;
iii. assigning a first descendant ID_{d} to said rough stone provided with table facet;
iv. processing said rough stone provided with table facet into a gemstone;
v. assigning a further descendant ID_{d} to said gemstone;
vi. tamperproof relating each descendant ID_{d} mutually and to the parental IDₚ.

2. Method for tamperproof relating at least two gemstones originating from one rough stone to said rough stone, comprising the steps of:
i. assigning to said rough stone a parental IDₚ;
ii. splitting, e.g. by cleaving or by sawing, said rough stone in at least two parts for further processing into at least two gemstones, thereby forming a table facet to each of the at least two parts;
iii. assigning a first descendant ID_{d} to each of said at least two parts;
iv. processing said at least two parts into at least two gemstones;
v. assigning a further descendant ID_{d} to each of said at least two gemstones;
vi. tamperproof relating each descendant ID_{d} mutually and to the parental IDₚ.

3. The method according to any of Claim 1 or 2, wherein said processing comprises the steps of bruting, blokking, further facetting and polishing.

4. The method according to Claim 3, wherein the assigning of a further descendant ID_{d} is subsequent to several processing steps.

5. The method according to any of Claim 3 or 4, wherein the assigning of a further descendant ID_{d} is subsequent to each processing step.

6. The method according to any of Claims 1 to 5, wherein said parental IDₚ and said first and further descendant ID_{d}'s are assigned by an incident radiation system.

7. The method according to Claim 6, wherein said radiation is chosen from the group comprising optical, electromagnetic and acoustic radiation.

8. The method according to Claim 7 wherein said radiation is optical.

9. The method according to any of Claims 1 to 8, wherein said parental IDₚ is assigned by an optical scanning system which is different from the optical scanning system in the assignment of the first and further descendant ID_{d}'s.

10. The method according to Claim 9, wherein said first descendant ID_{d}'s are assigned by a first optical scanning system which is equal to the optical scanning system in the assignment of the parental IDₚ and by a second optical scanning system which is equal to the optical scanning system in the assignment of the further descendant ID_{d}'s.

11. The method according to any of Claims 9 or 10, wherein said optical scanning system is laser scanning.

12. The method according to any of Claims 1 to 11, wherein said parental IDₚ is assigned by means of a laser scanning system producing a visual 3D output signal.

13. The method according to Claim 12, wherein said laser scanning system producing a visual 3D output signal is DiaExpert™.

14. The method according to any of Claims 1 to 11, wherein said further descendant ID_{d}'s are assigned by means of a laser scanning system producing an optical output of the internal refraction and reflection characteristics of the gemstones.

15. The method according to Claim 14, wherein said means of refraction and reflection of light arrays is Gemprint™.

16. Certificate of authenticity attesting the tamperproof relationship of a gemstone to its original rough stone comprising proof by the method according to any of claims 1 and 3 to 15 **characterized in**:
i. parental IDₚ of the rough stone;
ii. first descendant ID_{d} for the rough stone provided with table facet;
ii. further descendant ID_{d} for further processed rough stone provided with table facet; and
iii. attest of tamperproof relationship between each descendant ID_{d} mutually and the parental IDₚ.

17. Certificate of authenticity attesting the tamperproof relationship between at least two gemstones from the same rough stone to said rough stone comprising proof by the method according to any of claims 2 to 15 **characterized in**:
i. parental IDₚ of the rough stone;
ii. descendant ID_{d} for each of the at least two parts derived from splitting the rough stone;
iii. further descendant ID_{d} for each of the at least two parts derived from splitting the rough stone at some or each of the processing steps of the method according to any of claims 2 to 15; and
iv. attest of tamperproof relationship between the descendant ID_{d} of each of the at least two parts derived from the parental rough stone mutually and the parental IDₚ.

18. Kit comprising:
i. at least one gemstone derived from a rough stone, said at least one gemstone identified through a method according to any of claims 1 and 3 to 15 or through a method according to any of claims 2 to 15; and
ii. a certificate according to Claim 16 or according to Claim 17.

19. Use of a method according to any of claims 1 to 15 for proving genuineness of a gemstone.
